# EUROPÄISCHE PATENTANMELDUNG

(11) **EP 4 560 567 A1**
(43) Veröffentlichungstag der Anmeldung: **28.05.2025**
(21) Anmeldenummer: 23211250.8
(22) Anmeldetag: 21.11.2023
(51) Int. Cl.: G06T 5/77, A61B 1/00, G06V 10/00

(54) **EINRICHTUNG UND VERFAHREN ZUR BEOBACHTUNG EINES OPERATIONSFELDES**

(71) Anmelder: Erbe Elektromedizin GmbH, 72072 Tübingen (DE)
(72) Erfinder: MICHELMANN, Adrian, 72762 Reutlingen (DE); KALIM, Faisal, 72764 Reutlingen (DE)
(74) Vertreter: Rüger Abel Patentanwälte PartGmbB

(57) **Zusammenfassung**

Ein erfindungsgemäßes Verfahren und eine erfindungsgemäße Einrichtung zur Beobachtung einer Gewebeoberfläche eines Operationsgebiets sehen vor, dass im Sichtbereich des Operateurs liegende störende Objekte, wie beispielsweise Instrumente, Blutansammlungen oder dergleichen, aus dem Leitbild entfernt und gänzlich unsichtbar gemacht oder durch optische, nicht störende graphische Repräsentationen ersetzt werden. Dazu werden die im Vordergrund liegenden, störenden Objekte identifiziert und die durch sie verdeckte Gewebeoberfläche anhand von Ausschnitten aus älteren Bildern der Bilderfolge oder des Videostreams ersetzt.

Das Verfahren ist schnell, wenig rechenintensiv und ermöglicht dem Chirurgen eine verbesserte Kontrolle des Operationsvorgangs.

## Beschreibung

Gegenstand der Erfindung sind eine Einrichtung zur Beobachtung einer Gewebeoberfläche in einem Operationsgebiet bei einer Behandlung lebenden Gewebes, insbesondere bei einem chirurgischen Eingriff, sowie ein Verfahren zur Gewebebeobachtung.

Bei chirurgischen Eingriffen oder auch anderen Einwirkungen auf biologisches Gewebe ist es häufig erforderlich, dass der Behandler das Gewebe während der Behandlung visuell beurteilen kann. Beispielsweise muss bei endoskopischen Eingriffen, z.B. bei der Ablation von Magenschleimhaut, mittels Argon-Plasma-Koagulation beurteilt werden, wieviel Prozent der Magenschleimhaut bereits abladiert wurden. Bei der Ablationsbehandlung kann es sowohl zu einer Farbveränderung der Magenschleimhaut als auch zu Bewegungen derselben im Kamerabild kommen. Beispielsweise kann sich die Mukosa durch die Einwirkung des Plasmas zusammenziehen, womit sich auch die behandelte Fläche ändert. Eine zusätzliche Schwierigkeit kann durch Blutungen entstehen, die während der Behandlung auftreten. Austretendes Blut kann ausgedehnte Gewebeflächen verdecken, womit insbesondere auch der Ursprung der Blutung nicht mehr erkennbar ist. Außerdem können ein Endoskop oder auch vom Endoskop geführte Instrumente sowie sonstige im Sichtfeld des Endoskops Gegenstände Teile des Gewebes verdecken und somit für den Behandler unsichtbar machen.

Aus dem Stand der Technik sind Algorithmen zur Entfernung von Objekten aus Bildern bekannt. Dazu wird auf folgenden Stand der Technik verwiesen:

DE 10 2019 005 516 A1**,** WO 2013/144912 A1**,** US 2021/0374910 A1 sowie Rui Xu et A1 (2019), Deep Flow-Guided Video Inpainting, arXiv:1905.02884 und Dahun Kim et A1 (2019), Deep Video Inpainting, Proceedings of the IEEE/CVF Conference in Computer Vision and Pattern Recognition (CVPR), 2019, pp. 5792-5801 **und** Alisdair Newson et A1. Video Inpainting of Complex Scenes, arXiv:1503.05528**.**

Die WO 2013/144912 A1 beschreibt ein Bildverarbeitungssystem zur Verarbeitung von Bildern eines medizinischen mittels Röntgenstrahlung arbeitenden Bildgebungssystems, insbesondere bei der Angiographie. Dabei wird Kontrastmittel in Blutgefäße geleitet, womit bei der Bildgebung zwangsläufig auch das entsprechende Instrument, beispielsweise ein Katheter, aufgenommen wird. Weil dies vom Behandler als störend empfunden wird, sieht dieses System einen Baustein zur Erfassung der Instrumentenform sowie ein Bilderzeugungsmodul vor, der dazu eingerichtet ist, vom Instrument verursachte Artefakte zu erfassen und digital zu beseitigen. Dazu wird ein Bildinterpolationsprozess genutzt. Dazu ist bei der Bildgebung mittels Röntgenstrahlung allerdings die genaue Kenntnis der Position des Instruments sowie dessen Geometrie und seiner Charakteristika erforderlich.

Aus der US 2021/0374910 A1 ist ein Verfahren zur Ultraschallbildgebung und Bildverarbeitung mittels Kalman-Filter bekannt.

Die DE 10 2019 005 516 A1 offenbart das sogenannte Videoinpainting mittels verlässlichkeitsgewichteter Bewegungsschätzung. Dazu ist ein Videoeditor vorgesehen, der auf den Videoinhalt zugreift, in dem ein Zielbereich vorhanden ist, in dem ein oder mehrere Videoframes modifiziert werden sollen. Dazu berechnet der Videoeditor eine Bewegung für eine Begrenzung des Zielbereichs und interpoliert aus der Begrenzungsbewegung eine Zielbewegung eines Zielpixels innerhalb des Zielbereichs. Dadurch können Objekte aus einem Bild entfernt werden. Die Interpolation von Bilddaten birgt allerdings die Gefahr in sich, Bildinhalte zu generieren, die nicht mit dem vom entfernten Objekt verdeckten Bildinhalten übereinstimmen.

**Dahun Kim et. al.** offenbart zur Entfernung von Objekten aus Videosequenzen die Nutzung tiefer neuronaler Netzwerke zum sogenannten Videoinpainting. Zur Verbesserung bestehender Ansätze schlagen **Dahun Kim et al** die Nutzung von benachbarten Frames sowie die Bildsynthese noch unbekannter Bildbereiche vor.

Bei der endoskopischen Behandlung von Geweben kommt es auf die Echtzeitbildverarbeitung an, sodass der Behandler einen Videostream im Wesentlichen unverzögert angezeigt bekommt. Störende Objekte müssen deswegen in Echtzeit aus dem Bild entfernt werden, der Zugriff auf zukünftige Bilder (Frames) ist nicht möglich.

Daraus leitet sich die der Erfindung zugrundeliegende Aufgabe ab, eine Einrichtung sowie ein Verfahren zur Beobachtung einer Gewebeoberfläche in einem Operationsgebiet bei der Behandlung lebenden Gewebes anzugeben, wobei störende Objekte, insbesondere in Form und Größe zu Beginn der Behandlung unbekannte und sich gegebenenfalls erst während der Behandlung bildende Objekte sein können, wie beispielsweise Blutseen oder dergleichen.

Erfindungsgemäß wird die mittels mindestens einer Kamera aufgenommene Bilderfolge einem Identifikationsblock zugeführt, der darauf eingerichtet ist, Gebiete, die die Gewebeoberfläche wiedergeben, von Blindgebieten zu unterscheiden, in denen die Gewebeoberfläche von Vordergrundobjekten verdeckt ist. Vordergrundobjekte können Instrumente, Fremdkörper, Blutansammlungen ("Blutseen") oder ähnliches sein. Zur Erkennung von solchen Vordergrundobjekten und somit entsprechenden Blindbereichen können Objekterkennungsalgorithmen oder ein auf die Erkennung von Objekten trainiertes neuronales Netz sein.

Mit dieser Maßnahme gelingt es, solche Objekte auch dann zu erkennen, wenn ihre Form und Größe vor Beginn der Operation nicht genau bekannt sind. Beispielsweise kann ein neuronales Netz auf die Erkennung von Instrumenten und/oder auf die Erkennung von Blutansammlungen trainiert sein, wobei eine große Auswahl verschiedener Instrumente wie auch Blutseen verschiedener Farbe, Form und Größe als Trainingsmaterial genutzt werden können. Außerdem kann das neuronale Netz auf die Erkennung von Gewebeoberflächen trainiert sein, um somit Sichtgebiete und Blindgebiete sicher zu unterscheiden. Erfindungsgemäß wird weiter vorgesehen, von Vordergrundobjekten verdeckte Gewebegebiete, d.h. Blindgebiete durch Sichtgebiete älterer Bilder der Bilderfolge zu ersetzen. Zum Ersatz der Blindgebiete im aktuellen Bild werden vorzugsweise die jüngstmöglichen Bilder herangezogen. Es wird dazu in den älteren Bildern das jüngste Bild gesucht, in denen das betroffene Blindgebiete des aktuellen Bilds noch sichtbar war. Diese Bild wird genutzt, um den Bildausschnitt zu gewinnen, der das Blindgebiet des aktuellen Bilds im Wiedergabebild ersetzt. Auf diese Weise wird eine Interpolation von Bilddaten und somit die Gefahr der Darstellung von real nicht vorhandenen Gewebestrukturen vermieden.

In einer besonders zweckmäßigen Ausführungsform der Erfindung werden Bewegung und/oder Verformung der Gewebeoberfläche erfasst und auf dieser Basis Verzerrungskarten erstellt, die die Bewegung und/oder Verzerrung der Gewebeoberfläche von Bild zu Bild wiedergeben. Diese Verzerrungskarten können genutzt werden, um die aus älteren Bildern in Blindgebiete jüngerer Bilder zu übertragenden Abschnitte (Bildanteile) einer passenden Verzerrung zu unterziehen, sodass diese Abschnitte nahtlos in das Sichtgebiet des jüngeren Bildes passen.

Die Erfindung beruht auf der automatischen Identifikation der Blindgebiete durch Algorithmen oder neuronaler Netze. Zusätzlich kann eine Eingabeoption vorgesehen sein, um Objekte manuell als Vordergrundobjekte zu kennzeichnen, die aus dem dem Operateur anzuzeigenden Bild bzw. Bilderfolge zu entfernen sind. Weiter ist es möglich, anstelle der entfernten Objekte Ersatzobjekte anzuzeigen, die beispielsweise als Grafik lediglich die Kontur eines Instruments oder eines Blutsees oder eines sonstigen Vordergrundobjekts wiedergeben. Zum Beispiel können ein oder mehrere im Bild vorhandene Instrumente auf diese Weise oder auch auf andere Weise, zum Beispiel als halbdurchsichtige Objekte, dargestellt und so vom Behandler besser geführt werden.

Die Erfindung ermöglicht die Rekonstruktion verdeckter Bereiche in Echtzeit, sodass dem Anwender das hinter einem Objekt liegende Gewebe sichtbar gemacht wird, was eine bessere Navigation ermöglicht.

In der Zeichnung sind Ausführungsbeispiele der Erfindung veranschaulicht. Es zeigen:
Figur 1 einen Operateur bei der Arbeit mit der erfindungsgemäßen Einrichtung an einem Patienten, in schematisierter Darstellung
Figur 2 und 3 erfindungsgemäße Einrichtungen zur Beobachtung einer Oberfläche, im schematischen Blockschaltbild,
Figur 4 und 5 Algorithmen zur Bearbeitung einer Bilderfolge nach dem erfindungsgemäßen Verfahren in schematisierter Darstellung.

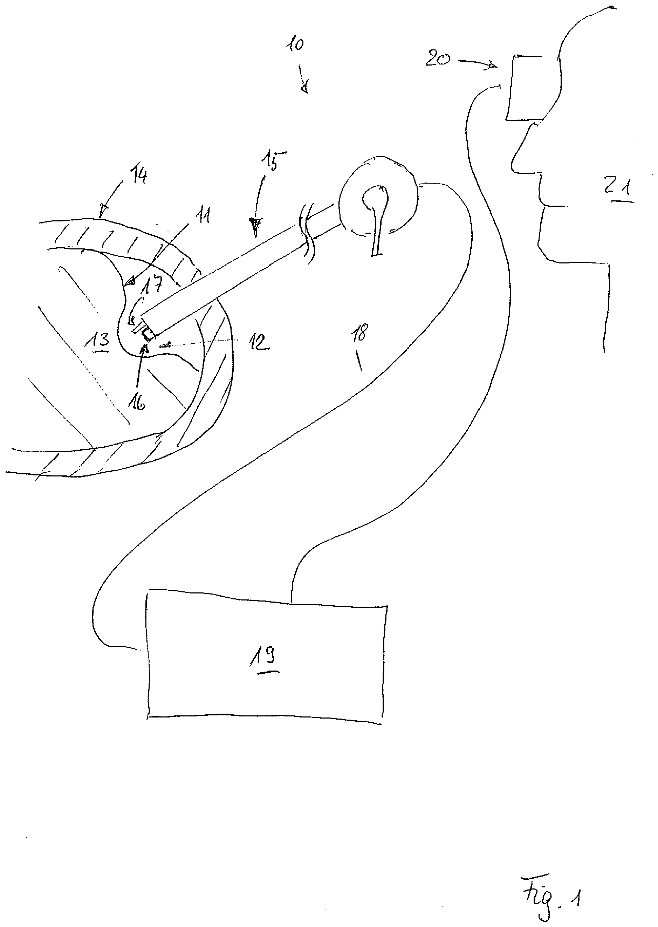

Figur 1 veranschaulicht eine erfindungsgemäße Einrichtung 10 zur Beobachtung einer Gewebeoberfläche 11, in einem Operationsgebiet 12 bei einer Behandlung lebenden Gewebes 13 eines Patienten 14. Das Operationsgebiet 12 kann sich in einer Körperhöhle eines Patienten befinden. Das Gewebe 13 kann Organgewebe sein, dessen Oberfläche 11, mittels einer von einem Endoskop 15 geführten oder an diesem vorgesehenen Kamera 16 beobachtbar ist. Die Kamera 16 kann eine einfache monokulare Kamera oder auch eine Stereokamera sein.

Mittels der Beobachtung des Operationsgebietes 12 durch die Kamera 16 kann die Wirkung eines Instruments 17 auf das Gewebe 13 sichtbar gemacht werden. Die Kamera 16 ist über eine geeignete Leitung 18 mit einer Bildverarbeitungseinrichtung 19 verbunden. Diese enthält eine Speichereinheit zur Abspeicherung von Bildern und generierten Verformungskarten. Die Bildverarbeitungseinrichtung 19 ist wiederum mit einer Bildwiedergabeeinrichtung 20 verbunden, die dem Operateur 21 das Operationsgebiet 12 veranschaulicht. Die Bildwiedergabeeinrichtung 20 kann eine Videobrille, ein Bildschirm oder dergleichen sein.

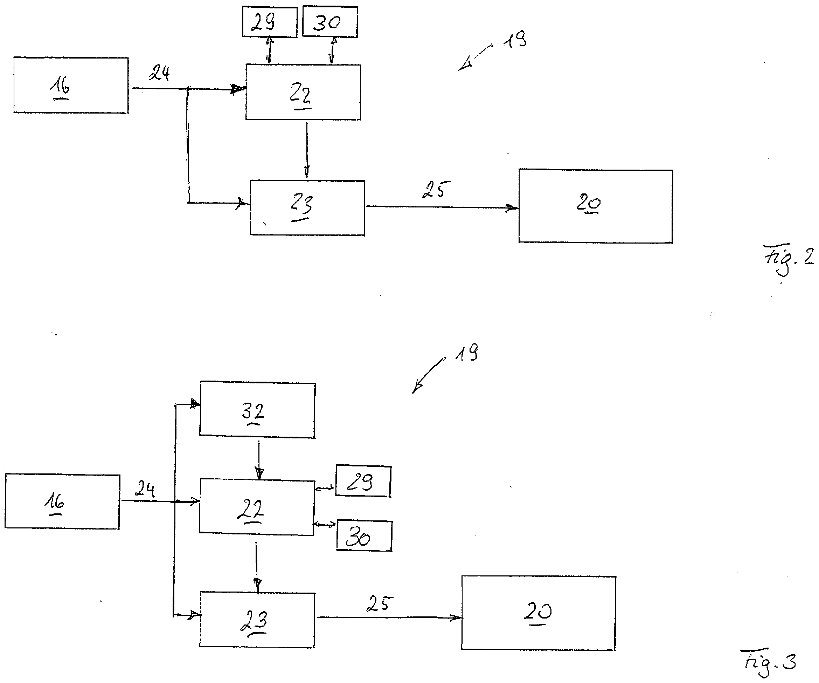

Figur 2 veranschaulicht eine einfache Ausführungsform der Bildverarbeitungseinrichtung 19 als Blockschaltbild. Der von der Kamera 16 gelieferte Videostream wird, wie die Pfeile andeuten, sowohl an einen Identifikationsblock 22 als auch an einem Einfügeblock 23 geliefert. Der Einfügeblock 23 dient dabei dazu, in den Bildern vorhandene Vordergrundobjekte durch Bilder des dahinterliegenden Gewebes zu ersetzen, sodass der Behandler 21 bestimmte Vordergrundobjekte, wie beispielsweise Instrumente oder dergleichen, nicht sieht.

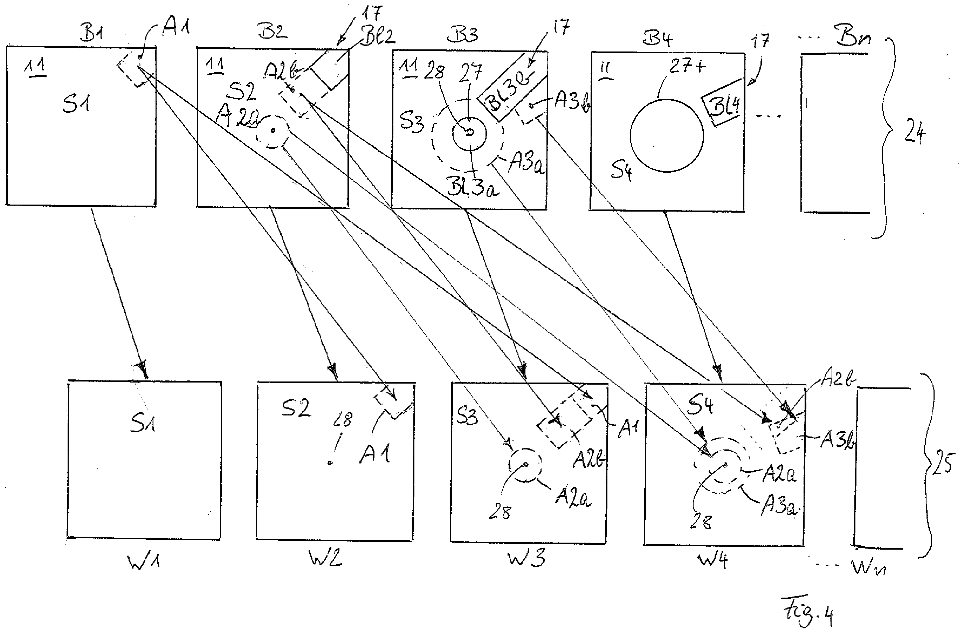

Zur Erläuterung des Aufbaus und Betriebs des Identifikationsblocks 22 und des Einfügeblocks 23 wird auf Figur 4 verwiesen. Dort ist die von der Kamera 16 gelieferte Bilderfolge 24 anhand von vier Beispielbildern B1, B2, B3, B4 ... Bn veranschaulicht. Aus dieser Bilderfolge 24 wird ein mittels der Bildwiedergabeeinrichtung 20 wiederzugebender Videostream 25 erzeugt, der aus Wiedergabebildern W1, W2, W3, W4 ... Wn besteht.

Die Erläuterung des Funktionsprinzips erfolgt hier anhand von vier Bildern B1 bis B4 und vier Wiedergabebildern W1 bis W4. Es versteht sich jedoch, dass die Anzahl der Bilder der Bilderfolge 24 und des Videostreams 25 unbestimmt ist. Auch kann die Anzahl der Bilder der Bilderfolge 24 von der Anzahl der Wiedergabebilder des Videostreams 25 abweichen.

In der Bilderfolge 24 ist die Gewebeoberfläche 11 erkennbar, die in Bild B1 das gesamte Bild einnimmt. Weder ist ein Instrument zu sehen, noch sonstige Vordergrundobjekte. Damit entspricht die gesamte Fläche des Bilds B1 einem Sichtgebiet S1. Dieses Sichtgebiet S1 kann ohne weitere Veränderung in das Wiedergabebild W1 übertragen werden. Dazu dient der Einfügeblock 23 gemäß Figur 2, der an dieser Stelle in das Sichtgebiet S1 nichts einfügt.

In dem zweiten Bild der Bilderfolge 24 ist ein Teil des Instruments 17 erkennbar, das sich vom Bildrand her in das Bild B2 geschoben hat. Das Instrument 17 verdeckt somit einen Abschnitt der Gewebeoberfläche 11 und erzeugt in dem Bild B2 somit einen Blindbereich Bl2, der einen Ausschnitt aus der ansonsten sichtbaren Gewebeoberfläche 11 verdeckt. Der entsprechende Sichtbereich S2 ist somit um den Blindbereich Bl2 verkleinert.

Der Identifikationsblock 22 ist dazu eingerichtet, den Blindbereich Bl2 in dem Bild B2 zu identifizieren. Der Identifikationsblock 22 teilt nun, wie aus Figur 2 hervorgeht, Lage und Größe des Blindbereichs Bl2 dem Einfügeblock 23 mit. Dieser wählt aus dem älteren Bild B1 einen in Lage und Größe mit dem Blindbereich Bl2 übereinstimmenden Ausschnitt A1 aus und fügt diesen in den Sichtbereich S2 in dem Bild W2 ein. Damit ist das Instrument 17 in dem Wiedergabebild W2 unsichtbar.

Im Verlauf der Bilderfolge 24 folgt das Bild B3, in dem das Instrument 17 weiter in Richtung Bildmitte vorgeschoben worden ist. Außerdem hat sich durch die Wirkung des Instruments 17 ein kleiner Blutsee 27 um einen Blutungsursprung 28 herum gebildet. Somit existieren in dem Bild B3 zwei Vordergrundobjekte, nämlich das Instrument 17 und der Blutsee 27. Beide verdecken teilweise die Sicht auf die Gewebeoberfläche 11 und stellen wiederum Blindbereiche Bl3a, Bl3b dar, die die erfindungsgemäße Einrichtung zu entfernen hat. Dazu identifiziert der Identifikationsblock 22 zunächst die Blindbereiche Bl3a, Bl3b, indem er die zugehörigen Objekte, nämlich das Instrument 17 und den Blutsee 27, mittels Mustererkennung, Bilderkennung, Farbunterscheidung oder durch geeignete Algorithmen erkennt. In Figur 2 sind dazu ein neuronales Netz 29 zur Instrumentenerkennung und ein neuronales Netz 30 zur Erkennung von Blutansammlungen durch entsprechende Blöcke veranschaulicht.

Nach der Erkennung der Blindbereiche Bl3a, Bl3b überträgt der Identifikationsblock 22 Lage und Größe der Blindbereiche Bl3a, Bl3b an den Einfügeblock 23. Dieser überträgt die in Lage und Größe übereinstimmenden Ausschnitte A1, A2 aus denjenigen jüngsten Bildern B1 und B2 in das Wiedergabebild W3, in denen das durch die Blindbereiche Bl3a, Bl3b verdeckte Gewebe noch sichtbar war. Durch diese Übertragung der Ausschnitte A1, A2a und A2b in das Wiedergabebild W3 werden der Blutsee 30 und das Instrument 17 in dem Wiedergabebild W3 unsichtbar. Der Blutungsursprung 28, der schon im Bild B2 vorhanden war, kann weiterhin angezeigt werden, wenn dieser zum Sichtbereich S1, S2 gehört.

Die weitere Bildverarbeitung erfolgt entsprechend. Bild 4 veranschaulicht den inzwischen deutlich vergrößerten Blutsee 27+ und das Instrument 17 in verlagerter Position. Die entsprechenden Ausschnitte A2a sowie A3a werden aus den älteren (jedoch jüngstmöglichen) Bildern B2 und B3 in das aktuelle Wiedergabebild B4 übertragen. Entsprechendes gilt für den geänderten von dem Instrument 17 hervorgerufenen Blindbereich B4, der zum Teil aus dem Ausschnitt A2b aus dem Bild B2 und zum Teil aus dem Ausschnitt A3b aus dem Bild B3 ergänzt wird. Wiederum sind in dem Wiedergabebild W4 die Vordergrundobjekte, nämlich der Blutsee 27+ und das Instrument 17, unsichtbar.

Anstelle der vollkommenen Entfernung des Blutsees 27, 27+ und des Instruments 17 aus dem Videostream 25 kann auch ein graphischer Ersatz derselben im Videostream 25 eingeblendet werden, beispielsweise, um Lage und Wirkung des Instruments 17 sowie die weitere Ausbreitung des Blutsees 27, 27+ für den Operateur weiterhin wahrnehmbar zu machen.

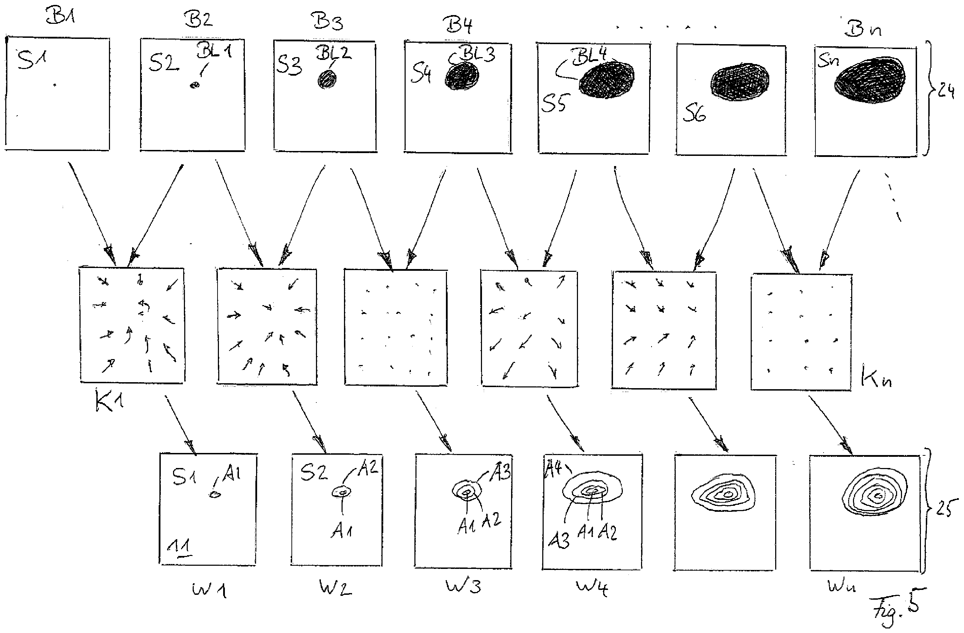

Figur 3 veranschaulicht eine erweiterte Ausführungsform der erfindungsgemäßen Bildverarbeitungseinrichtung 19, die dazu eingerichtet ist, Bewegungen und Verzug der Gewebeoberfläche 11 im Operationsgebiet 12 zu berücksichtigen. Diese Bildverarbeitungseinrichtung 19 enthält zusätzlich zu dem Identifikationsblock 22 und dem Einfügeblock 23 einen Erfassungsblock 32, der anhand von in den Figuren nicht weiter veranschaulichten Gewebemerkmalen, zum Beispiel anhand der Textur der Gewebeoberfläche 12, Verzug und Verlagerung der Gewebeoberfläche 12 in entsprechenden Karten K1 bis Kn ermittelt. In Figur 5 sind diese Verzerrungskarten K1 bis Kn schematisch als Vektorfelder veranschaulicht. Sie können durch muskuläre Reaktionen, Gewebeveränderungen, durch Kamerabewegung oder dergleichen verursacht und bestimmt sein.

Die Karten K1 bis Kn werden von dem Erfassungsblock 32 an den Identifikationsblock 22 und/oder den Einfügeblock 23 übertragen. Die Sichtbereiche S1 bis Sn werden von dem Einfügeblock 23 unmittelbar in die Wiedergabebilder W1 bis Wn übernommen. Die durch den anwachsenden Blutsee 27, 27+ usw. entstehenden Blindgebiete werden durch entsprechende Ausschnitte A1, A2, A3, A4 usw. ersetzt, wobei sie anhand der Verzerrungskarten K1 bis Kn entsprechend der Verzerrung der Gewebeoberfläche 11 im Sichtgebiet S1, S2, S3, S4 usw. angepasst werden.

Zusätzlich zu dem Ersatzes der Blindgebiete durch Ausschnitte aus älteren Bildern ist es auch möglich, den Ausschnitt so über das Blindgebiet zu legen, dass der im Blindgebiet vorhandene Gegenstand (z.B. Instrument, Blutsee oder dergleichen) noch abgeschwächt sichtbar bleibt. Auch kann der im Blindgebiet vorhandene Gegenstand durch eine grafische Repräsentation ersetzt werden. Eine solche Repräsentation kann z.B. ein Umriss eines Instruments, eine das Instrument oder einen anderen Gegenstand repräsentierende Punktewolke oder dergleichen mehr sein.

In der Beschreibung der Ausführungsform nach Figur 2 und 4 sowie nach Figur 3 und 5 ist davon ausgegangen worden, dass alle Blindgebiete durch Ausschnitte aus Sichtgebieten der Bilderfolge 24 ersetzt werden. Es ist jedoch auch möglich, in dem Videostream 25 vorhandene bereits ersetzte Ausschnitte A1, A2, A3 usw. jeweils aus dem vorigen Bild W1, W2, W3 des Videostreams 25 zu übernehmen, sodass kein erneuter Rückgriff auf Bilder der Bilderfolge 24 erforderlich ist, sondern lediglich diejenigen Blindgebiete durch Bildausschnitte aus Sichtgebieten der Bilderfolge 24 ersetzt werden müssen, die in dem Videostream 25 noch nicht ersetzt worden sind.

Ein erfindungsgemäßes Verfahren und eine erfindungsgemäße Einrichtung zur Beobachtung einer Gewebeoberfläche 11 eines Operationsgebiets 12 sehen vor, dass im Sichtbereich S des Operateurs liegende störende Objekte, wie beispielsweise Instrumente 17, Blutansammlungen 27 oder dergleichen, aus dem Wiedergabebild W (Livebild) entfernt und gänzlich unsichtbar gemacht oder durch optische, nicht störende graphische Repräsentationen ersetzt werden. Dazu werden die im Vordergrund liegenden störenden Objekte identifiziert und die durch sie verdeckte Gewebeoberfläche anhand von Ausschnitten A aus älteren Bildern B der Bilderfolge 24 oder des Videostreams 25 ersetzt.

Das Verfahren ist schnell, wenig rechenintensiv und ermöglicht dem Chirurgen eine verbesserte Kontrolle des Operationsvorgangs.

### Bezugszeichen:

- 10: Einrichtung
- 11: Gewebeoberfläche
- 12: Operationsgebiet
- 13: Gewebe
- 14: Patient
- 15: Endoskop
- 16: Kamera
- 17: Instrument
- 18: Leitung
- 19: Bildverarbeitungseinrichtung
- 20: Bildwiedergabeeinrichtung
- 21: Operateur
- 22: Identifikationsblock
- 23: Einfügeblock
- 24: Bilderfolge
- B: Bild der Bilderfolge 24
- B1 ... B4: Bilder der Bilderfolge 24
- Bl: Blindbereich allgemein
- Bl1: Blindbereiche in Bild B1
- Bl2a, Bl2b: Blindbereiche in Bild B1, B2
- 25: Videostream
- W: Wiedergabebild des Videostreams 25
- W1 ... W4: Wiedergabebilder des Videostreams 25
- S: Sichtgebiet in einem Bild B
- S1 ... S4: Sichtgebiete
- A: Ausschnitt aus dem Sichtbereich S eines Bilds B
- A1: Ausschnitt aus dem Sichtbereich S1
- A2: Ausschnitt aus dem Sichtbereich S2
- A3: Ausschnitt aus dem Sichtbereich S3
- 27, 27+: Blutsee
- 28: Blutungsursprung
- 29, 30: neuronale Netze
- B4: Blindbereich
- 32: Erfassungsblock
- K1 ... Kn: Verzerrungskarten

## Patentansprüche

1. Einrichtung (10) zur Beobachtung einer Gewebeoberfläche (11) in einem Operationsgebiet (12) bei einer Behandlung lebenden Gewebes (13),
mit mindestens einer Kamera (16) zur Aufnahme einer Folge (24) von Bildern (B1 ... Bn) von der zu behandelnden Gewebeoberfläche (11),
mit einem Identifikationsblock (22), der zur Identifikation von Sichtgebieten (S1 ... Sn) in den Bildern (B1 ... Bn) eingerichtet ist, die die Gewebeoberfläche (11) wiedergeben, und der zur Identifikation von Blindgebieten (Bl1 ... Bln) in den Bildern (B1 ... Bn) eingerichtet ist, die zu Vordergrundobjekten (17, 27) gehören, die die Gewebeoberfläche (11) verdecken,
mit einem Einfügeblock (23), der dazu eingerichtet ist, zum Ersatz von Blindgebieten (Bl1 ... Bln) des aktuellen Bildes (B1 ... Bn) wenigstens einen Abschnitt (A1 ... An) eines Sichtgebiets (S1 ... Sn) aus einem älteren Bild (B) der Bilderfolge (24) auszuwählen, um es in das Blindgebiet (Bl1 ... Bln) einzufügen.

2. Einrichtung nach Anspruch 1, weiter **gekennzeichnet durch** einen Erfassungsblock (32), der zur Erfassung von Bewegung Verformung oder visueller Änderung der Gewebeoberfläche (11) und zur Erstellung von Verzerrungskarten (K1 ... Kn) eingerichtet ist.

3. Einrichtung nach Anspruch 1 und 2, **dadurch gekennzeichnet, dass** der Einfügeblock dazu eingerichtet ist, den aus einem älteren Bild (B) ausgewählten Abschnitt (A1 ... An) des Sichtgebietes (S1 ... Sn) vor dem Einfügen den Verzerrungskarten (K1 ... Kn) entsprechend zu transformieren.

4. Einrichtung nach einem der vorstehenden Ansprüche,
**dadurch gekennzeichnet, dass** der Identifikationsblock (22) zur Identifikation der Blindgebiete (Bl1 ... Bln) einen Objekterkennungsalgorithmus oder ein neuronales Netz zur Objekterkennung aufweist.

5. Einrichtung nach einem der vorstehenden Ansprüche,
**dadurch gekennzeichnet, dass** der Identifikationsblock (22) eine Eingabeoption zur manuellen Objektidentifikation aufweist.

6. Verfahren zur Beobachtung einer Gewebeoberfläche (11) in einem Operationsgebiet bei einer Behandlung lebenden Gewebes, bei dem
eine Folge (24) von Bildern (B1 ... Bn) von der zu behandelnden Gewebeoberfläche (11) aufgenommen wird,
in den Bildern (B1 ... Bn) Sichtgebiete (S1 ... Sn) identifiziert werden, die die Gewebeoberfläche (11) wiedergeben,
in den Bildern (B1 ... Bn) Blindgebiete (Bl1 ... Bln) identifiziert werden, die zu Vordergrundobjekten (17, 27) gehören, die die Gewebeoberfläche (11) verdecken,
Blindgebiete (Bl1 ... Bln) des aktuellen Bildes (B) durch wenigstens einen Abschnitt (A1 ... An) eines Sichtgebiets (S1 ... Sn) aus einem älteren Bild (B) der Bilderfolge (24) ersetzt werden.

7. Verfahren nach Anspruch 6, **dadurch gekennzeichnet, dass** außerdem Bewegung, Verformung oder visuelle Änderung der Gewebeoberfläche (11) erfasst werden.

8. Verfahren nach Anspruch 7, **dadurch gekennzeichnet, dass** auf Basis der erfassten Bewegungen, Verformungen oder visuellen Änderungen Verzerrungskarten (K1 ... Kn) erstellt werden.

9. Verfahren nach Anspruch 7 oder 8, **dadurch gekennzeichnet, dass** der aus einem älteren Bild (B) ausgewählte Abschnitt (A1 ... An) des Sichtgebietes (S1 ... Sn) vor dem Einfügen den Verzerrungskarten (K1 ... Kn) entsprechend der erfassten Bewegung, Verformung oder visuellen Änderung verformt wird.

10. Verfahren nach einem der vorstehenden Ansprüche 6 bis 9, **dadurch gekennzeichnet, dass** die Blindgebiete (Bl1 ... Bln) mittels eines Objekterkennungsalgorithmus ermittelt werden.

11. Verfahren nach einem der vorstehenden Ansprüche 6 bis 9, **dadurch gekennzeichnet, dass** die Blindgebiete (Bl1 ... Bln) mittels eines neuronalen Netzes zur Objekterkennung ermittelt werden.

12. Verfahren nach einem der vorstehenden Ansprüche 10 oder 11, **dadurch gekennzeichnet, dass** die Blindgebiete (Bl1 ... Bln) zusätzlich manuell identifiziert werden.
